Europäisches Patentamt

European Patent Office

Office européen des brevets

(19)

(11) Publication number : **0 463 030 B1**

(12) **EUROPEAN PATENT SPECIFICATION**

(45) Date of publication of patent specification :
28.07.93 Bulletin 93/30

(51) Int. Cl.⁵ : **A61K 7/42**

(21) Application number : **90904618.7**

(22) Date of filing : **16.03.90**

(86) International application number :
**PCT/EP90/00433**

(87) International publication number :
**WO 90/11067 04.10.90 Gazette 90/23**

(54) SUNSCREEN COMPOSITIONS.

(30) Priority : **18.03.89 GB 8906258**

(43) Date of publication of application :
**02.01.92 Bulletin 92/01**

(45) Publication of the grant of the patent :
**28.07.93 Bulletin 93/30**

(84) Designated Contracting States :
**AT BE CH DE DK ES FR GB IT LI LU NL SE**

(56) References cited :
**DE-A- 3 824 999**
**Adverse effects of sunscreens in photosensitive patients, by P.M. Farr et-al, The Lancet , Feb 25 1989, p 429 - 430**
**Seifen-Oele-Fette-Wachse, volume 113, no.20, 10 December 1987, (Augsburg, DE) ; W.-D. GRIEBLER:"Titandioxid - ein anorganischer Rohstoff in der Kosmetik", pages 765-771, see page 766, paragraph 2.1.3.: Transparentes TiO2 als UV-Absorber ; figures 4-3**

(56) References cited :
**PATENT ABSTRACTS OF JAPAN, volume 11, no. 371 (C-462)(2818), 3 December 1987, 29 June 1987**
**PATENT ABSTRACTS OF JAPAN, volume 13, no. 156 (C-585)(3504), 14 April 1989, & JP-A-63310810**
**PATENT ABSTRACTS OF JAPAN, volume 13, no. 11 (C-558)(3359), 11 January 1989, & JP-A-63218615**
**PATENT ABSTRACTS OF JAPAN, volume 13, no. 461 (C-645)(3809), 18 October 1989, & JP-A-01180819**

(73) Proprietor : **The Boots Company PLC**
**1 Thane Road West**
**Nottingham Nottinghamshire NG2 3AA (GB)**

(72) Inventor : **ELSOM, Nicola, Anne**
**120 Burton Road Melton Mowbray**
**Leicestershire LE13 1DL (GB)**
Inventor : **GALLEY, Edward**
**11 Peebles Road Newark**
**Nottinghamshire NG24 4RW (GB)**

(74) Representative : **Filler, Wendy Anne**
**The Boots Company plc Patents Department**
**R4 Pennyfoot Street**
**GB-Nottingham NG2 3AA (GB)**

Jouve, 18, rue Saint-Denis, 75001 PARIS

EP 0 463 030 B1

EP 0 463 030 B1

**Description**

The present invention relates to sunscreen compositions. The term "sunscreen" is used herein to encompass sunscreening compositions such as moisturisers, day creams, tanning lotions and sunblockers which are intended for topical application to provide protection against the sun's rays or other sources of ultraviolet (UV) radiation.

Heretofore sunscreen compositions have been prepared either as oil-in-water or water-in-oil emulsions containing organic sunscreen agents which could be formulated equally successfully in either of the above emulsion systems. More recently sunscreen compositions have been proposed which contain as the sunscreening agent, titanium dioxide particles. Generally small titanium dioxide particles, for example 15 nm, are employed in such compositions because larger particles are associated with whiteness which greatly reduces their utility in sunscreen compositions.

W.D. Griebler in "Titandioxid - ein anorganischer Rohstoff in der Kosmetic" [Seifen - Öle - Fette - Wachse, Vol.113 (1987) 10 Dez. No.20, Augsburg, Germany, pp 765/771] describes the properties of titanium dioxide and its potential for use in cosmetics compositions such as sunscreen compositions. It is stated that the finest materials are often hard to disperse, and that addition absorption phenomena may take place at the surface of titanium dioxide particles.

GB-A-2217987 (corresponding to DE-A-3824999) in the name of The Boots Company PLC discloses a sunscreen composition in the form of a water-in-oil emulsion which comprises inter alia from 0.5 to 30% by weight of titanium dioxide having a mean primary particle size of less than 100 nm.

It has been reported (Farr and Diffey - The Lancet, February 25, 1989, pages 429-430) that the sunscreens known heretofore offer adequate protection against the ultraviolet B wavelengths but that they offer little or no protection against ultraviolet A wavelengths. To adequately protect the skin from damage on exposure to sunlight which contains both ultraviolet A and ultraviolet B radiation, a sunscreen is required which provides protection against both of these types of ultraviolet radiation. This is particularly so in the case of people whose skin is particularly sensitive. There is, for example, a photosensitive skin disorder, known as polymorphic light eruption, in which an itchy, papular, erthematous rash develops on skin exposed to sunlight, usually within hours of exposure. Polymorphic light eruption has been shown by experimental and epidemiological evidence to be caused by exposure to ultraviolet A wavelengths. Patients suffering from this disorder have attempted to protect themselves by the use of commercially available sunscreens but this can cause the disorder to become more severe. The use of a sunscreen which reduces the exposure only to ultraviolet B radiation encourages the patient to remain exposed to sunlight for a longer period of time because the patient is less aware of the exposure they are receiving. The exposure to ultraviolet A radiation is therefore increased and the danger of the onset of polymorphic light eruption is also increased. Patients suffering from polymorphic light eruption have therefore been advised not to use sunscreen products but instead to reduce their exposure to sunlight. This is obviously inconvenient to the patient.

It is known to provide opaque reflectant sunscreens which reflect visible and ultraviolet B radiation but which provide poor protection against ultraviolet A radiation. These reflectant sunscreens appear opaque when used on the skin. Whilst such opaque compositions have found acceptance on the ski slopes they are not acceptable under most other circumstances where protection is required but the user does not wish their use of a sunscreen to be apparent.

The object of the present invention is therefore to provide a sunscreen composition which provides adequate protection against both ultraviolet A and ultraviolet B radiation and which is cosmetically acceptable.

The present invention provides a sunscreen composition which comprises 5 to 30% by weight of microfine titanium dioxide particles in combination with a cosmetically acceptable diluent or carrier, said composition being characterised in that the titanium dioxide comprises a blend of 10 to 70% titanium dioxide having a mean primary particle size of about 15 nm and 30 to 90% of at least one further grade of titanium dioxide having a mean primary particle size of between about 30 nm and about 50 nm and in that, in use, it is substantially transparent on the skin and in that the ratio of UVA to UVB absorption when measured at 370 and 310 nm is in the range of 0.25 to 0.6, preferably 0.3 to 0.6.

In this specification "microfine" titanium dioxide is that which has a mean primary particle size (mpps) of between 1 and 100 nm and more preferably between 15 and 50 nm. The titanium dioxide may have an anatase, rutile or amorphous structure. The particles may be uncoated or may be provided with a coating of an aluminium compound such as aluminium oxide, aluminium stearate, aluminium laurate, or a phospholipid such as lecithin.

Suitable grades of titanium dioxide for use in the compositions of the present invention are available commercially. For example Teikoku Kako Co Ltd supply titanium dioxide having a mean primary particle size of around 15 nm under the trade designations MT100T and MT150W. The former of these products has the titanium dioxide particles coated with aluminium stearate whereas the latter is uncoated. Uncoated titanium dioxides having mean primary particle sizes of around 35 nm and around 50 nm are available from the above company under the trade designations MT500B and MT600B respectively.

Each commercially available titanium dioxide grade contains a distribution of particle sizes around the mean primary particle size but none alone provides the desired characteristics of the sunscreen compositions according to the invention since the spread of sizes is too small. The microfine titanium dioxide of use in the compositions according to the invention comprises a blend of different particle sizes between 1 and 100 nm, preferably between about 10 nm and about 50 nm. The blend of titanium dioxide comprises a mixture of at least two, preferably three different grades of microfine titanium dioxide each grade having a different mean primary particle size (mpps) within the range of 1 to 100 nm, preferably between about 10 and about 50 nm. In a preferred embodiment, the blend of titanium dioxide comprises 10 to 70%, preferably 15 to 65%, most preferably 20 to 60% titanium dioxide having a mpps of about 15 nm and 30 to 90%, preferably 35 to 85%, most preferably 40 to 80% of at least one further grade of titanium dioxide having a mpps of between about 30 nm and about 50 nm.

More particularly the blend of titanium dioxide comprises 10 to 70% of titanium dioxide having a mean primary particle size of around 15 nm; 10 to 60%, preferably 20 to 50%, most preferably 25 to 40% of titanium dioxide having a mean primary particle size of around 35 nm; and 10 to 60%, preferably 10 to 50%, most preferably 15 to 40% of titanium dioxide having a mean primary particle size of around 50 nm.

The total amount of titanium dioxide present in any particular sunscreen composition according to the present invention depends on the use for which the composition is intended. The so-called sunblock compositions which are intended to prevent substantially all of the sun's rays reaching the skin may require levels of titanium dioxide as high as 30%. Particularly preferred sunscreen compositions according to the invention comprise 5 to 20% by weight of titanium dioxide.

The compositions of the present invention may be formulated in any of the known ways for such products e.g. milks, lotions, creams, gels, sticks or aerosols. The preferred formulation types are water-in-oil or oil-in-water emulsions such as lotions or creams.

A typical oil-in-water emulsion comprises:-
a) 5 to 30% by weight of a blend of microfine titanium dioxide particles;
b) 5 to 40% by weight of an oil phase;
c) 1 to 20% by weight of an emulsifier; and
d) at least 30% by weight of an aqueous phase.
A typical water-in-oil emulsion comprises:
a) 5 to 30% by weight of a blend of microfine titanium dioxide particles;
b) 5 to 50% by weight of an oil phase;
c) 1 to 15% by weight of an emulsifier; and
d) at least 30% by weight of an aqueous phase.
The oil phase of the oil phase dispersions and the water-in-oil and oil-in-water emulsions of the present invention may comprise for example:
a) hydrocarbon oils such as paraffin or mineral oils;
b) waxes such as beeswax or paraffin wax;
c) natural oils such as sunflower oil, apricot kernel oil, shea butter or jojoba oil;
d) silicone oils such as dimethicone, cyclomethicone or cetyldimethicone;
e) fatty acid esters such as isopropyl palmitate or isopropyl myristate;
f) fatty alcohols such as cetyl alcohol or stearyl alcohol; or
g) mixtures thereof, for example, the blend of waxes available commercially under the trade name Cutina (Henkel).
In preferred water-in-oil compositions of the present invention the oil phase comprises 5 to 40%, more preferably 10 to 30% by weight of the composition. In preferred oil-in-water compositions of the present invention the oil phase comprises 5 to 30%, more preferably 10 to 20% by weight of the composition.

The emulsifiers used may be any emulsifiers known in the art for use in water-in-oil or oil-in-water emulsions. It has been found that particularly effective water-in-oil and oil-in-water sunscreen compositions can be prepared by using an emulsifier or mixture of emulsifiers selected from known cosmetically acceptable emulsifiers which include:
a) sesquioleates such as sorbitan sesquioleate, available commercially for example under the trade name Arlacel 83 (ICI), or polyglyceryl-2-sesquioleate;
b) ethoxylated esters of derivatives of natural oils such as the polyethoxylated ester of hydrogenated castor oil available commercially for example under the trade name Arlacel 989 (ICI);
c) silicone emulsifiers such as silicone polyols available commercially for example under the registered trade name ABIL WS08 (Th. Goldschmidt AG);

d) anionic emulsifiers such as fatty acid soaps e.g. potassium stearate and fatty acid sulphates e.g. sodium cetostearyl sulphate available commercially under the trade name Dehydag (Henkel);

e) ethoxylated fatty alcohols, for example the emulsifiers available commercially under the trade name Brij (ICI);

f) sorbitan esters, for example the emulsifiers available commercially under the trade name Span (ICI);

g) ethoxylated sorbitan esters, for example the emulsifiers available commercially under the trade name Tween (ICI);

h) ethoxylated fatty acid esters such as ethoxylated stearates, for example the emulsifiers available commercially under the trade name Myrj (ICI);

i) ethoxylated mono-, di-, and tri-glycerides, for example the emulsifiers available commercially under the registered trade name Labrafil (Alfa Chem.);

j) non-ionic self-emulsifying waxes, for example the wax available commercially under the trade name Polawax(Croda);

k) ethoxylated fatty acids, for example, the emulsifiers available commercially under the registered trade name Tefose (Alfa Chem.); or

l) mixtures thereof.

The amount of emulsifier present in the water-in-oil compositions of the present invention is preferably in the range 2 to 10%. Preferred water-in-oil emulsifiers are anionic or non-ionic emulsifiers. The amount of emulsifier present in the oil-in-water compositions of the present invention is preferably in the range 1 to 15%, more preferably 2 to 15%. Preferred oil-in-water emulsifiers include ethoxylated fatty acids and alcohols, ethoxylated stearates and ethoxylated triglycerides and mixtures thereof.

The compositions of the present invention may additionally comprise other components which will be well known to those skilled in the art. These include, for example, emolients such as isopropyl myristate or triglycerides of fatty acids e.g. lauric triglyceride or capric/caprylic triglyceride, such as the triglyceride available commercially under the registered trade name Migliol 810 (Huls UK); moisturisers such as D-panthenol; humectants such as glycerin or 1,3-butylene glycol; antioxidants such as DL-$\alpha$-tocopherylacetate or butylated hydroxytoluene; emulsion stabilising salts such as sodium chloride, sodium citrate or magnesium sulphate; film formers to assist spreading on the surface of the skin such as alkylated polyvinylpyrrolidone e.g. available commercially under the registered trade name Antaron (GAF); thickeners such as acrylic acid polymers e.g. available commercially under the registered trade name Carbopol (B.F. Goodrich) or modified celluloses e.g. hydroxyethylcellulose available commercially under the registered trade name Natrosol (Hercules); preservatives such as bronopol, sodium dehydroacetate, polyhexamethylenebiguanide hydrochloride, isothiazolone or diazolidinylurea; sequestering agents such as EDTA salts; perfumes and colourings.

Other sunscreening agents may be incorporated into the compositions of the present invention. Examples of suitable further sunscreening agents include a) p_aminobenzoic acids, their esters and derivatives (for example, 2-ethylhexyl p_-dimethylaminobenzoate), b) methoxycinnamate esters (for example, 2-ethylhexyl p_-methoxycinnamate, 2-ethoxyethyl p_-methoxycinnamate or $\alpha,\beta$-di-(p_-methoxycinnamoyl)-$\alpha$'-(2-ethylhexanoyl)glycerin, c) benzophenones (for example oxybenzone), d) dibenzoylmethanes and e) salicylate esters. Any additional sunscreening agent is present in an amount from 0.1 to 10% by weight of the composition.

The compositions of the present invention described hereinbefore provide adequate protection against both UVA and UVB radiation in a composition which is aesthetically pleasing to the user. The compositions of the present invention are of particular value to patients suffering from polymorphic light eruption. Compositions according to the invention which contain a relatively high concentration, for example 5 to 30%, titanium dioxide and provide a high protection against UVB and UVA radiation are particularly advantageous.

The opacity or whiteness of compositions may be estimated by trained observers by subjective visual analysis of the relative transparency of the composition spread onto skin. Surprisingly, all the compositions according to the invention exemplified hereinafter were substantially transparent on the skin. However, a quantitative assessment of the opacity or whiteness of compositions may be obtained as follows.

A circular area of diameter 23 mm was marked on the forearm of a volunteer and the surface reflectance of that area measured with a Minolta chromometer. 10 $\mu$l of product was then applied to the test site and spread evenly with a glass rod. The product was left to dry for 2-3 minutes and then the surface reflectance of the area measured again. Each product was tested on several volunteers. The difference between the mean surface reflectance (L-value) before and after product application gives a quantitative indication of the whiteness of the product.

Products giving a mean L-value increase of greater than 5.5 were considered to be cosmetically unacceptable. Preferred compositions according to the invention had a mean L-value increase of less than about 5.0.

The ratio of UVA to UVB absorption at 370 nm and 310 nm respectively was measured by the method described in full by Diffey and Robson in J. Soc. Cosmet. Chem., 40, 127-133 (1989) but which is summarised below.

Adhesive tape commercially available from 3M-Company, USA, under the registered trade name Transpore was used as the medium to which the product was applied. This tape is UV-transparent and has a textured surface which has been found to distribute sunscreen compositions in a way similar to the uneven topography of human skin. A piece of tape (75 mm x 65 mm) was placed over the input slit of an f 3.4 grating monochromator (Applied Photophysics Ltd, UK), attached to a photomultiplier detector, and illuminated with "white" light from a 75 W xenon arc source. The intensity of the radiation transmitted through the tape was measured by recording the photocurrent in 5 nm steps from 290-400 nm.

100 µl of sunscreen (2.0 mg/cm$^2$) was evenly distributed onto the tape surface, the tape replaced over the monochromator input slit and transmission measurements were repeated. For each product the transmittance was determined at 12 separate subsites within the 77 x 65 mm application site. The ratio of absorption at 370 and 310 nm was then calculated from the mean measurement at each wavelength.

The invention is illustrated by the following Examples 1 to 5 which are given as illustrations only. Comparative Examples A to C form no part of the present invention.

The titanium dioxide used in the examples was coated with lecithin in the following way.

Preparation of lecithin-coated titanium dioxide

1) Isopropyl palmitate          3 g
2) Lecithin (sold under the trade name P Centrolex)          1.5 g
3) Microfine titanium dioxide          95.5 g

Components 1 and 2 were mixed together and heated to 85°C. This solution was slowly added to component 3 with mixing on a high speed powder mixer (Papenmeiyer). The resulting mixture was mixed for a further 15 minutes using the Papenmeiyer. The powder was then milled twice using a hammer mill (Mikropul Ducon) to produce a fine, free-flowing powder of lecithin-coated titanium dioxide.

Different grades of titanium dioxide may be separately coated with lecithin prior to blending or may be pre-blended and then the blend of titanium dioxide particles coated with lecithin.

Example 1

A sunscreen composition was prepared from the following components:-

|  | | | % w/w |
|---|---|---|---|
| 1) | A cyclodimethicone and silicone copolyol mixture (sold under the registered trade designation Dow Corning 3225C) | | 12 |
| 2) | Cyclomethicone (sold under the registered trade designation Dow Corning 345) | | 15 |
| 3) | Cetyl dimethicone (sold under the registered trade designation ABIL B9801) | | 5 |
| 4) | Sorbitan sesquioleate (sold under the trade name Arlacel 83) | | 3 |
| 5) | Butylated hydroxytoluene | | 0.05 |
| 6) | Titanium Dioxide having a mean primary particle size (mpps) of 15 nm (sold under the trade designation MT150W) – lecithin coated | | 2 |
| 7) | Titanium Dioxide having a mpps of 35 nm (sold under the trade designation MT500B) – lecithin coated | | 5 |
| 8) | Titanium Dioxide having a mpps of 50 nm (sold under the trade designation MT600B) – lecithin coated | | 3 |
| 9) | Sodium citrate | | 4 |
| 10) | Bronopol | | 0.02 |
| 11) | Sodium dehydroacetate | | 0.15 |
| 12) | Water | | to 100% |

Components 4 and 5 were heated together at 70°C and then mixed into components 1 to 3 using a high shear mixer/homogeniser (Silverson). Components 6, 7 and 8 (titanium dioxide) were dispersed into the oil phase (components 1 to 5) using the Silverson mixer/homogeniser. Components 9, 10 and 11 were dissolved in the water and the aqueous phase was slowly added to the oil phase with stirring and the mixer homogenised to viscosity with the Silverson mixer/homogeniser.

The ratio of WA to UVB absorption measured at 370 nm and 310 nm was 0.47

Comparative Examples A, B and C

Components 6, 7 and 8 of Example 1 were replaced by single grades of titanium dioxide to give the formulations of Comparative Examples A, B and C. The titanium dioxide used, the resultant ratios of UVA and UVB absorption measured at 370 nm and 310 nm, and the quantitative measure of whiteness (L-value) of the formulations were as shown in Table 1 (Example 1 included for comparison).

TABLE 1

| Example | Titanium dioxide mpps/% | | | UVA UVB Ratio | L-value increase |
|---|---|---|---|---|---|
| | 15 nm | 35 nm | 50 nm | | |
| 1 | 2 | 5 | 3 | 0.47 | 4.7 |
| A | 10 | - | - | 0.21 | 3.1 |
| B | - | 10 | - | 0.37 | 5.6 |
| C | - | - | 10 | 0.62 | 5.9 |

Examples 2 and 3

The titanium dioxide (components 6, 7 and 8) of the formulation described in Example 1 was replaced by different concentrations of titanium dioxide. The concentrations and resultant ratios of UVA and UVB absorption measured at 370 nm and 310 nm were as shown in Table 2 (Example 1 included for comparison).

Table 2

| Example | Titanium dioxide mpps/% | | | UVA UVB ratio |
|---|---|---|---|---|
| | 15 nm MT150W | 35 nm MT500B | 50 nm MT600B | |
| 1 | 2.0 | 5.0 | 3.0 | 0.47 |
| 2 | 1.0 | 2.0 | 2.0 | 0.49 |
| 3 | 3.0 | 5.0 | 7.0 | 0.54 |

Example 4

The titanium dioxide (components 6, 7 and 8) of the formulation described in Example 1 was replaced by 7% titanium dioxide having a mpps of 15 nm (sold under the trade designation MT 100T), 4% titanium dioxide having a mpps of 35 nm (sold under the trade designation MT500B) and 2.4% titanium dioxide having a mpps of 50 nm (sold under the trade designation MT600T), all lecithin coated.

The ratio of UVA to UVB absorption measured at 370 nm and 310 nm was 0.3.

7

Example 5

The titanium dioxide (components 6, 7 and 8) of the formulation described in Example 1 was replaced by 9% titanium dioxide having a mpps of 15 nm (sold under the trade designation MT100T), 4% titanium dioxide having a mpps of 35 nm (sold under the trade designation MT500B) and 2.4% titanium dioxide having a mpps of 50 nm (sold under the trade designation MT600T), all lecithin coated.

The ration of UVA to UVB absorption measured at 370 nm and 310 nm was 0.25.

## Claims

### Claims for the following Contracting States : AT, BE, CH, DE, DK, FR, GB, IT, LI, LU, NL, SE

1. A sunscreen composition which comprises 5 to 30% by weight of microfine titanium dioxide particles in combination with a cosmetically acceptable diluent or carrier, said composition being characterised in that the titanium dioxide comprises a blend of 10 to 70% titanium dioxide having a mean primary particle size of about 15 nm and 30 to 90% of at least one further grade of titanium dioxide having a mean primary particle size of between about 30 nm and about 50 nm and in that, in use, it is substantially transparent on the skin and in that the ratio of UVA to UVB absorption when measured at 370 and 310 nm is in the range of 0.25 to 0.6.

2. A sunscreen composition as claimed in claim 1 wherein the blend of titanium dioxide comprises 10 to 70% titanium dioxide having a mean primary particle size of about 15 nm; 10 to 60% of titanium dioxide having a mean primary particle size of around 35 nm; and 10 to 60% of titanium dioxide having a mean primary particle size of around 50 nm.

3. A sunscreen composition as claimed in claim 1 or claim 2 wherein the mean L-value increase is less than 5.5.

4. A sunscreen composition as claimed in any one of the preceding claims which is an oil-in-water emulsion comprising:
   a) 5 to 30% by weight of a blend of microfine titanium dioxide particles;
   b) 5 to 40% by weight of an oil phase;
   c) 1 to 20% by weight of an emulsifier; and
   d) at least 30% by weight of an aqueous phase.

5. A sunscreen composition as claimed in any one of claims 1 to 3 which is an water-in-oil emulsion comprising:
   a) 5 to 30% by weight of a blend of microfine titanium dioxide particles;
   b) 5 to 50% by weight of an oil phase;
   c) 1 to 15% by weight of an emulsifier; and
   d) at least 30% by weight of an aqueous phase.

6. A sunscreen composition as claimed in any one of the preceding claims wherein the titanium dioxide particles are coated with lecithin.

7. The use of a composition as claimed in any one of the preceding claims as a sunscreen.

8. The use of a composition as claimed in any one of claims 1 to 6 in the treatment of polymorphic light eruption.

9. The use of a composition as claimed in any one of claims 1 to 6 in the manufacture of a medicament for the treatment of polymorphic light eruption.

### Claims for the following Contracting State : ES

1. A method of preparing a sunscreen composition by combining 5 to 30% by weight of microfine titanium dioxide particles with a cosmetically acceptable diluent or carrier, said composition being characterised in that the titanium dioxide comprises a blend of 10 to 70% titanium dioxide having a mean primary particle size of about 15 nm and 30 to 90% of at least one further grade of titanium dioxide having a mean primary particle size of between about 30 nm and about 50 nm and in that, in use, it is substantially transparent on the skin and in that the ratio of WA to UVB absorption when measured at 370 and 310 nm is in the range of 0.25 to 0.6.

2. A method as claimed in claim 1 wherein the blend of titanium dioxide comprises 10 to 70% titanium dioxide having a mean primary particle size of about 15 nm; 10 to 60% of titanium dioxide having a mean primary particle size of around 35 nm; and 10 to 60% of titanium dioxide having a mean primary particle size of around 50 nm.

3. A method as claimed in claim 1 or claim 2 wherein the mean L-value increase is less than 5.5.

4. A method as claimed in any one of the preceding claims to provide a composition which is a oil-in-water emulsion comprising:
   a) 5 to 30% by weight of a blend of microfine titanium dioxide particles;
   b) 5 to 40% by weight of an oil phase;
   c) 1 to 20% by weight of an emulsifier; and
   d) at least 30% by weight of an aqueous phase.

5. A method as claimed in any one of claims 1 to 3 to provide a composition which is a water-in-oil emulsion comprising:
   a) 5 to 30% by weight of a blend of microfine titanium dioxide particles;
   b) 5 to 50% by weight of an oil phase;
   c) 1 to 15% by weight of an emulsifier; and
   d) at least 30% by weight of an aqueous phase.

6. A method as claimed in any one of the preceding claims wherein the titanium dioxide particles are coated with lecithin.

7. The use of a composition as defined in any one of the preceding claims as a sunscreen.

8. The use of a composition as defined in any one of claims 1 to 6 in the treatment of polymorphic light eruption.

9. The use of a composition as defined in any one of claims 1 to 6 in the manufacture of a medicament for the treatment of polymorphic light eruption.


**Patentansprüche**

**Patentansprüche für folgende Vertragsstaaten : AT, BE, CH, DE, DK, FR, GB, IT, LI, LU, NL, SE**

1. Sonnenschutzmittel, das 5 bis 30 Gew.-% mikrofeine Titandioxidteilchen in Kombination mit einem kosmetisch verträglichen Verdünner oder Trägerstoff enthält,
   **dadurch gekennzeichnet,** daß das Titandioxid eine Mischung aus 10 bis 70 % Titandioxid mit einer mittleren Primärteilchengröße von etwa 15 nm und 30 bis 90 % mindestens einer weiteren Sorte Titantioxid mit einer mittleren Primärteilchengröße zwischen etwa 30 nm und etwa 50 nm umfaßt,
   daß es bei der Verwendung im wesentlichen transparent auf der Haut ist und
   daß das Verhältnis von UVA- zu UVB-Absorption gemessen bei 370 und 310 nm im Bereich von 0,25 zu 0,6 liegt.

2. Sonnenschutzmittel nach Anspruch 1, in dem die Titandioxidmischung 10 bis 70 % Titandioxid mit einer mittleren Primärteilchengröße von etwa 15 nm, 10 bis 60 % Titandioxid mit einer mittleren Primärteilchengröße von etwa 35 nm und 10 bis 60 % Titandioxid mit einer mittleren Primärteilchengröße von etwa 50 nm enthält.

3. Sonnenschutzmittel nach Anspruch 1 oder 2, in dem die mittlere L-Wert-Steigerung weniger als 5,5 beträgt.

4. Sonnenschutzmittel nach einem der vorstehenden Ansprüche in Form einer Öl-in-Wasser-Emulsion, die
   a) 5 bis 30 Gew.-% einer Mischung mikrofeiner Titandioxidteilchen,
   b) 5 bis 40 Gew.-% Ölphase,
   c) 1 bis 20 Gew.-% Emulgator und
   d) mindestens 30 Gew.-% wäßrige Phase
   enthält.

5. Sonnenschutzmittel nach einem der Ansprüche 1 bis 3 in Form einer Wasser-in-Öl-Emulsion, die
   a) 5 bis 30 Gew.-% einer Mischung aus mikrofeinen Titandioxidteilchen,
   b) 5 bis 50 Gew.-% Ölphase
   c) 1 bis 15 Gew.-% Emulgator und
   d) mindestens 30 Gew.-% wäßrige Phase
   enthält.

6. Sonnenschutzmittel nach einem der vorstehenden Ansprüche, in dem die Titandioxidteilchen mit Lecithin beschichtet sind.

7. Verwendung einer Zusammensetzung nach einem der vorstehenden Ansprüche als Sonnenschutzmittel.

8. Verwendung einer Zusammensetzung nach einem der Ansprüche 1 bis 6 bei der Behandlung polysymptomatischer, durch Licht verursachter Ausschläge.

9. Verwendung einer Zusammensetzung nach einem der Ansprüche 1 bis 6 bei der Herstellung eines Medikaments zur Behandlung polysymptomatischer, durch Licht verursachter Ausschläge.

**Patentansprüche für folgenden Vertragsstaat : ES**

1. Verfahren zur Herstellung eines Sonnenschutzmittels durch Kombination von 5 bis 30 Gew.-% mikrofeiner Titandioxidteilchen mit einem kosmetisch verträglichen Verdünner oder Trägerstoff,
   **dadurch gekennzeichnet,** daß das Titandioxid eine Mischung aus 10 bis 70 % Titandioxid mit einer mittleren Primärteilchengröße von etwa 15 nm und 30 bis 90 % mindestens einer weiteren Sorte Titandioxid mit einer mittleren Primärteilchengröße zwischen etwa 30 nm und etwa 50 nm umfaßt,
   daß es bei der Verwendung im wesentlichen transparent auf der Haut ist und
   daß das Verhältnis von UVA- zu UVB-Absorption gemessen bei 370 und 310 nm im Bereich von 0,25 zu 0,6 liegt.

2. Verfahren nach Anspruch 1, bei dem die Titandioxidmischung 10 bis 70 % Titandioxid mit einer mittleren Primärteilchengröße von etwa 15 nm, 10 bis 60 % Titandioxid mit einer mittleren Primärteilchengröße von etwa 35 nm und 10 bis 60 % Titandioxid mit einer mittleren Primärteilchengröße von etwa 50 nm enthält.

3. Verfahren nach Anspruch 1 oder 2, bei dem die mittlere L-Wert-Steigerung weniger als 5,5 beträgt.

4. Verfahren nach einem der vorstehenden Ansprüche, um eine Zusammensetzung in Form einer Öl-in-Wasser-Emulsion zur Verfügung zu stellen, die
   a) 5 bis 30 Gew.-% einer Mischung mikrofeiner Titandioxidteilchen,
   b) 5 bis 40 Gew.-% Ölphase,
   c) 1 bis 20 Gew.-% Emulgator und
   d) mindestens 30 Gew.-% wäßrige Phase
   enthält.

5. Verfahren nach einem der Ansprüche 1 bis 3, um eine Zusammensetzung in Form einer Wasser-in-Öl-Emulsion, zur Verfügung zu stellen, die
   a) 5 bis 30 Gew.-% einer Mischung aus mikrofeinen Titandioxidteilchen,
   b) 5 bis 50 Gew.-% Ölphase
   c) 1 bis 15 Gew.-% Emulgator und
   d) mindestens 30 Gew.-% wäßrige Phase
   enthält.

6. Verfahren nach einem der vorstehenden Ansprüche, bei dem die Titandioxidteilchen mit Lecithin beschichtet sind.

**7.** Verwendung einer Zusammensetzung wie in einem der vorstehenden Ansprüche definiert als Sonnenschutzmittel.

**8.** Verwendung einer Zusammensetzung wie in einem der Ansprüche 1 bis 6 definiert bei der Behandlung polysymptomatischer, durch Licht verursachter Ausschläge.

**9.** Verwendung einer Zusammensetzung wie in einem der Ansprüche 1 bis 6 definiert bei der Herstellung eines Medikaments zur Behandlung polysymptomatischer , durch Licht verursachter Ausschläge.


**Revendications**

**Revendications pour les Etats contractants suivants : AT, BE, CH, DE, DK, FR, GB, IT, LI, LU, NL, SE**

**1.** Composition de protection solaire, qui comprend 5 à 30 % en poids de particules microscopiques de dioxyde de titane en association avec un diluant ou support acceptable du point de vue cosmétique, ladite composition étant caractérisée en ce que le dioxyde de titane comprend un mélange de 10 à 70 % de dioxyde de titane ayant un diamètre principal moyen des particules d'environ 15 nm et de 30 à 90 % d'au moins une autre qualité de dioxyde de titane ayant un diamètre principal moyen des particules compris entre environ 30 nm et environ 50 nm et en ce que, lors de son utilisation, elle est principalement transparente sur la peau et le rapport d'absorption UVA:UVB, mesuré à 370 et à 310 nm, se situe dans la plage de 0,25 à 0,6.

**2.** Composition de protection solaire suivant la revendication 1, dans laquelle le mélange de dioxyde de titane comprend 10 à 70 % de dioxyde de titane ayant un diamètre principal moyen des particules d'environ 15 nm ; 10 à 60 % du dioxyde de titane ayant un diamètre principal moyen des particules de l'ordre de 35 nm ; et 10 à 60 % du dioxyde de titane ayant un diamètre principal moyen des particules de l'ordre de 50 nm.

**3.** Composition de protection solaire suivant la revendication 1 ou la revendication 2, dans laquelle l'augmentation moyenne de la valeur L est inférieure à 5,5.

**4.** Composition de protection solaire suivant l'une quelconque des revendications précédentes, qui est une émulsion huile-dans-eau comprenant :
a) 5 à 30 % en poids d'un mélange de particules microscopiques de dioxyde de titane ;
b) 5 à 40 % en poids d'une phase d'huile ;
c) 1 à 20 % en poids d'un émulsionnant ; et
d) au moins 30 % en poids d'une phase aqueuse.

**5.** Composition de protection solaire suivant l'une quelconque des revendications 1 à 3, qui est une émulsion eau-dans-huile comprenant :
a) 5 à 30 % en poids d'un mélange de particules microscopiques de dioxyde de titane ;
b) 5 à 50 % en poids d'une phase d'huile ;
c) 1 à 15 % en poids d'un émulsionnant ; et
d) au moins 30 % en poids d'une phase aqueuse.

**6.** Composition de protection solaire suivant l'une quelconque des revendications précédentes, dans laquelle les particules de dioxyde de titane sont revêtues de lécithine.

**7.** Utilisation d'une composition suivant l'une quelconque des revendications précédentes comme protection solaire.

**8.** Utilisation d'une composition suivant l'une quelconque des revendications 1 à 6 dans le traitement d'une éruption polymorphe due à la lumière.

**9.** Utilisation d'une composition suivant l'une quelconque des revendications 1 à 6 dans la fabrication d'un médicament destiné au traitement d'une éruption polymorphe due à la lumière.

**Revendications pour l'Etat contractant suivant : ES**

1. Procédé de préparation d'une composition de protection solaire, par mélange de 5 à 30 % en poids de particules microscopiques de dioxyde de titane avec un diluant ou support acceptable du point de vue cosmétique, ladite composition étant caractérisée en ce que le dioxyde de titane comprend un mélange de 10 à 70 % de dioxyde de titane ayant un diamètre principal moyen des particules d'environ 15 nm et de 30 à 90 % d'au moins une autre qualité de dioxyde de titane ayant un diamètre principal moyen des particules compris entre environ 30 nm et environ 50 nm et en ce que, lors de son utilisation, elle est principalement transparente sur la peau et le rapport d'absorption UVA:UVB, mesuré à 370 et à 310 nm, se situe dans la plage de 0,25 à 0,6.

2. Procédé suivant la revendication 1, dans lequel le mélange de dioxyde de titane comprend 10 à 70 % de dioxyde de titane ayant un diamètre principal moyen des particules d'environ 15 nm ; 10 à 60 % du dioxyde de titane ayant un diamètre principal moyen des particules de l'ordre de 35 nm ; et 10 à 60 % du dioxyde de titane ayant un diamètre principal moyen des particules de l'ordre de 50 nm.

3. Procédé suivant la revendication 1 ou la revendication 2, dans lequel l'augmentation moyenne de la valeur L est inférieure à 5,5.

4. Procédé suivant l'une quelconque des revendications précédentes, pour l'obtention d'une composition qui est une émulsion huile-dans-eau comprenant :
   a) 5 à 30 % en poids d'un mélange de particules microscopiques de dioxyde de titane ;
   b) 5 à 40 % en poids d'une phase d'huile ;
   c) 1 à 20 % en poids d'un émulsionnant ; et
   d) au moins 30 % en poids d'une phase aqueuse.

5. Procédé suivant l'une quelconque des revendications 1 à 3 pour l'obtention d'une composition qui est une émulsion eau-dans-huile comprenant :
   a) 5 à 30 % en poids d'un mélange de particules microscopiques de dioxyde de titane ;
   b) 5 à 50 % en poids d'une phase d'huile ;
   c) 1 à 15 % en poids d'un émulsionnant ; et
   d) au moins 30 % en poids d'une phase aqueuse.

6. Procédé suivant l'une quelconque des revendications précédentes, dans lequel les particules de dioxyde de titane sont revêtues de lécithine.

7. Utilisation d'une composition suivant l'une quelconque des revendications précédentes comme protection solaire.

8. Utilisation d'une composition suivant l'une quelconque des revendications 1 à 6 dans le traitement d'une éruption polymorphe due à la lumière.

9. Utilisation d'une composition suivant l'une quelconque des revendications 1 à 6 dans la fabrication d'un médicament destiné au traitement d'une éruption polymorphe due à la lumière.